# FASCICULE DE BREVET EUROPEEN

(11) **EP 0 412 920 B1**
(45) Date de publication et mention de la délivrance du brevet: **31.05.1995**
(21) Numéro de dépôt: 90440067.8
(22) Date de dépôt: 18.07.1990
(51) Int. Cl.: A61K 51/08, A61K 49/00

(54) **Composition pour le diagnostic et/ou le traitement, notamment du cancer**
Zusammensetzung für die Diagnose und Behandlung insbesondere von Krebs
Diagnostic or therapeutic composition, especially for cancer

(30) Priorité: 08.08.1989 FR 8910797
(43) Date de publication de la demande: 13.02.1991
(73) Titulaire: UNIVERSITE DE ROUEN, F-76130 Mont-Saint-Aignan (FR)
(72) Inventeur: Girard, Nicole, F-76000 Rouen (FR); Delpech, Bertrand, F-76410 Saint-Aubin lès Elbeuf (FR); Courel, Marie-Noelle, F-76000 Rouen (FR); Chomant, Jean, F-76770 Malaunay (FR)

(56) Documents cités:
- WO-A-89/07456
- STN INTERNATIONAL (KARLSRUHE) BASE C.A. & CHEMICAL ABSTRACTS, vol.109, no. 25, résumé no. 225918z, Columbus, Ohio, US; G. GALLI et al.: "Aradiopharmaceutical for the study of the liver: technetium-99m-asialo-orosomucoid. I: Radiochemical and animal distribution studies",& J. NUCL. MED. ALLIED SCI., 32(2), 110-16
- STN INTERNATIONAL (KARLSRUHE) BASE C.A. & CHEMICAL ABSTRACTS, vol.
- 97, no. 5, résumé no. 36939e, Columbus, Ohio, US; A. DELPECH et al.:"Hyaluronectin in normal human skin and in basal cell carcinoma",& BR. J. DERMATOL., 106(5), 561-8
- BIOCHEMISTRY, vol. 25, no. 18, 9 septembre 1986, pages 4979-4983, AmericanChemical Society, Washington, DC, US; T.J. KUNICKI et al.: "Covalent attachmentof sulfhydryl-specific, electron spin, resonance spin-labels to fab' fragmentsof murine monoclonal antibodies that recognize human platelet membraneglycoproteins. Development of membrane protein specific spin probes"

## Description

La présente invention a pour objet l'utilisation à des fins de diagnostic et/ou de traitement, notamment du cancer, d'une glycoprotéine particulière, l'hyaluronectine.

On sait les difficultés attachées tant au diagnostic qu'au traitement du cancer, et l'importance que revêt en particulier la mise au point d'une méthode de diagnostic efficace autorisant un traitement aussi précoce que possible de la maladie.

On a déjà proposé, à cette fin, une méthode scintigraphique faisant appel à l'utilisation d'anticorps monoclonaux chargés d'isotopes radioactifs. Toutefois les inconvénients liés à cette méthode sont nombreux, tenant pour une part à l'affinité relativement faible des anticorps monoclonaux pour les antigènes cancéreux, pour une autre part à leur étroite spécificité vis-à-vis desdits antigènes, et pour une dernière part aux réactions immunologiques inévitables de l'organisme humains vis-à-vis d'anticorps provenant d'animaux, le plus souvent des souris. A ces inconvénients s'ajoute celui créé par le fait que l'antigène qui fixe ces anticorps n'est pas toujours représenté dans les métastases cancéreuses mais seulement dans la tumeur primitive, en sorte que cette méthode ne permet pas de localiser avec certitude la totalité des sites cancéreux d'un organisme.

Le problème se posait donc de mettre au point une méthode qui ne présente aucun de ces inconvénients et qui permette un diagnostic sûr et complet en autorisant la localisation de tous les sites cancéreux d'un organisme humain, et ce sans risque de réactions immunologiques.

La présente invention a pour but d'offrir une solution au problème ainsi posé en proposant une composition utilisable pour le diagnostic du cancer qui joint à l'avantage de l'efficacité et de la fiabilité celui de l'universalité, étant applicable à la détection de toutes les tumeurs cancéreuses, quels que soient leur type histologique, leur localisation et leur nature, primitive ou métastatique.

La présente invention a également pour but de proposer une composition utilisable pour le traitement de certains types de tumeurs cancéreuses, notamment les tumeurs intracérébrales.

La présente invention a ainsi pour objet une composition utilisable à des fins de diagnostic et/ou de traitement, notamment du cancer, cette composition se caractérisant essentiellement en ce qu'elle renferme une glycoprotéine particulière, l'hyaluronectine.

La présente invention est en effet fondée sur cette double observation, d'une part que l'acide hyaluronique est présent dans toutes les tumeurs cancéreuses, sans exception, et d'autre part qu'il présente une affinité élevée pour l'hyaluronectine, laquelle est par ailleurs capable de fixer un produit tel qu'un radioisotope, susceptible d'avoir une action thérapeutique ou d'être détecté par des moyens d'exploration externes comme la scintigraphie.

L'acide hyaluronique est un glycosaminoglycane qui constitue un composant des tissus conjonctifs lâches et inflammatoires, et qui se retrouve en des proportions importantes dans les tissus cancéreux, où il est associé à la matrice extracellulaire.

L'hyaluronectine, glycoprotéine présente dans l'encéphale et le cordon ombilical de l'homme et des animaux, présente la propriété avantageuse d'être d'une taille notablement inférieure à celle des anticorps monoclonaux, puisque possédant une masse moléculaire de 68 000, en sorte que sa pénétration dans les tissus se fait facilement.

L'acide hyaluronique se trouvant associé en proportions importantes à la matrice extracellulaire des tissus cancéreux, est aisément accessible à l'hyaluronectine qui se fixe sur lesdits tissus préférentiellement aux tissus conjonctifs sains dans lesquels sa concentration est faible ou nulle.

Marquée par un radioisotope approprié émetteur de rayons γ comme l'indium, l'hyaluronectine peut ainsi permettre la localisation des sites cancéreux d'un organisme soit en imagerie externe, par scintigraphie, soit en repérage peropératoire.

Par ailleurs, une charge convenablement dosée d'un radioisotope émetteur de rayons β fixée sur l'hyaluronectine peut permettre un traitement très efficace de certaines tumeurs radio-sensibles, en particulier des tumeurs intracérébrales.

L'hyaluronectine mise en oeuvre dans la composition selon l'invention peut être isolée du cerveau d'animaux ou de cordons ombilicaux humains par passage d'un extrait acide sur un absorbant d'acide hyaluronique couplé à une combinaison aminohexyl-sépharose, suivi d'une élution acide.

Selon un premier mode de réalisation de l'invention, applicable au diagnostic du cancer, l'hyaluronectine est marquée à l'aide d'un élément détectable par des moyens d'exploration externes avant d'être injectée dans l'organisme humain sous forme de solution aqueuse.

Le marquage de l'hyaluronectine peut s'effectuer au moyen d'un radioisotope détectable par scintigraphie, mais aussi au moyen d'un élément paramagnétique, tel que le gadolinium, détectable par résonance magnétique nucléaire (RMN).

Dans le cas d'un radioisotope, on peut mettre en oeuvre n'importe quel élément émetteur de rayons γ approprié au radio-diagnostic, comme l'indium 111, le technétium 99, l'iode 123 ou l'iode 131, cette énumération n'étant pas limitative.

La fixation de l'élément choisi sur l'hyaluronectine peut s'effectuer par toute méthode de couplage connue appropriée à la fixation d'un tel élément sur une molécule de protéine.

Ainsi, dans le cas de l'indium ou du technétium, on fait appel à un agent chélatant, l'acide diéthylènetriamine pentaacétique (DTPA), dont on réalise le couplage avec l'hyaluronectine, le DTPA libre étant éliminé par chromatographie de perméation sur gel avant fixation de l'indium ou du technétium.

Dans le cas de l'iode, la fixation peut s'opérer au moyen de la chloramine T ou de l'iodogène.

Le marquage de l'hyaluronectine s'effectue à l'aide des quantités d'élément radioactif les plus faibles possible susceptibles d'être détectées par les moyens d'exploration habituels. On peut ainsi réaliser ce marquage de manière que l'hyaluronectine porte une charge de radioactivité n'excédant pas 10 microcuries par microgramme, et de préférence comprise entre 1 et 5 microcuries par microgramme.

Selon un second mode de réalisation de l'invention, applicable au traitement du cancer, l'hyaluronectine est chargée d'un isotope radioactif émetteur de rayons β utilisé dans la radiothérapie du cancer, comme par exemple l'iode 131.

Dans ce second mode de réalisation, l'hyaluronectine sert de vecteur au moyen thérapeutique qu'elle présente aux différents sites cancéreux.

Ce moyen de traitement est particulièrement approprié à la thérapie des tumeurs intracérébrales, qui présentent la propriété de ne pas donner lieu à des métastases et d'être particulièrement riches en acide hyaluronique accessible.

Dans ce second mode de réalisation, le radioélément émetteur de rayons β fixé sur l'hyaluronectine doit être mis en oeuvre à des doses permettant de délivrer à la tumeur une dose de quelques dizaines de grays, ordre de grandeur des doses délivrées en radiothérapie externe dans le traitement des cancers.

L'exemple qui suit illustre la présente invention appliquée au diagnostic du cancer, étant bien entendu que cet exemple ne présente aucun caractère limitatif vis-à-vis de l'invention.

### EXEMPLE

On isole l'hyaluronectine à partir d'un cerveau d'agneau par passage d'un extrait acide sur un adsorbant d'acide hyaluronique (AH) couplé à de l'AH - sépharose, suivi d'une élution acide.

On réalise ensuite le couplage de la protéine en appréciant la quantité de DTPA couplé sur une aliquote que l'on marque à l'indium et que l'on chromatographie sur feuille de silice, le DTPA libre étant éliminé sur Séphadex G 100.

On contrôle l'activité de l'hyaluronectine (HN) par test, sur coupe de tissu, de son affinité pour l'AH, puis on la marque à l'indium 111, à raison de 5 » Ci / » g et on l'injecte à des souris.

On a choisi à cet effet des souris DBA/2 porteuses d'un carcinome mammaire greffé de génération en génération, auxquelles on a injecté 6 » g d'HN marquée. Les souris ont été scintigraphiées à J2, J3 et J4, des comptages d'activité dans les zones de tumeur ou d'organes délimitées sur les images étant effectués à ces différents temps, conduisant aux résultats rassemblés dans le tableau ci-après.

| | J2 (3 cas) | J3 (4 cas) | J4 (5 cas) |
|---|---|---|---|
| Tu/Foie | 0,66-0,96-0,64 | 0,86-0,89 | 0,58-0,70-0,60 |
| | | 0,82-0,50 | 0,47-0,60 |
| Tu/Tête | 3,42-4,59-3,08 | 4,88-4,74 | 2,56-4,67-3,38 |
| | | 3,70-3,50 | 3,40-2,95 |
| Tu/Abdomen | 3,33-4,37-3,42 | 2,75-3,68 | 1,85-3,85-2,84 |
| | | 3,57-1,90 | 2,56-2,27 |

A l'exception du foie et des reins, on a constaté que la fixation d'HN dans la tumeur a toujours été supérieure à sa fixation dans les autres organes, l'activité au niveau du foie et des reins correspondant à des fixations non spécifiques du radiotracteur (voies d'élimination).

Par ailleurs, des comptages d'activité d'organes (foie, rate, rein, cerveau, muscle, poumon, coeur, sang) prélevés sur les souris sacrifiées ont été effectués à J2, J4 ET J6 et ont montré que la radioactivité n'était pas retrouvée dans le cerveau, le coeur et les poumons. Les index de localisation de la protéine marquée dans la tumeur (Tu) par rapport au sang ont été les suivants :

| | J2 (1 cas) | J4 (2 cas) | J6 (3 cas) |
|---|---|---|---|
| Tu/Sang | 6,5 | 11,7 | 11 |
| | | 5,1 | 8,7 |
| | | | 11,8 |

Les résultats, tant en imagerie qu'en étude de biodistribution, montrent que l'hyaluronectine injectée se fixe préférentiellement dans la tumeur, permettant sa détection et/ou son traitement.

La composition selon l'invention présente l'avantage, déjà exposé, de permettre la détection et la localisation de toutes les tumeurs cancéreuses, primitives ou métastatiques, quel que soit leur type histologique, ce qui constitue un progrès considérable par rapport aux méthodes connues, et de permettre en outre le traitement efficace de certains types de tumeurs, notamment des tumeurs intracérébrales.

A cet avantage s'ajoutent ceux liés d'une part à la stabilité de l'hyaluronectine, qui peut être conservée plusieurs mois à 4°C et résiste quelques minutes à 100°C, et d'autre part à la simplicité de sa préparation, laquelle ne soulève pas de problème juridique ou éthique, l'hyaluronectine pouvant être isolée à partir d'encéphales d'animaux ou de cordons ombilicaux.

A cet égard, l'utilisation d'une préparation faite à partir de cordon ombilical humain permet d'éviter en toute certitude les réactions immunologiques, constantes avec les anticorps monoclonaux de souris.

Il convient par ailleurs de signaler que la présente invention peut trouver une application endehors de la cancérologie, notamment en rhumatologie et dans tous les cas où apparaît une réaction du stroma conjonctif, entraînant l'accessibilité de l'acide hyaluronique à l'hyaluronectine.

## Revendications

1. Composition utilisable pour le diagnostic et/ou le traitement, notamment du cancer, caractérisée en ce qu'elle consiste en une solution aqueuse injectable d'une glycoprotéine, l'hyaluronectine, sur laquelle est fixé un élément radioactif.

2. Composition selon la revendication 1 utilisable pour le diagnostic, notamment du cancer, caractérisée en ce que l'élément radioactif fixé sur l'hyaluronectine est un radioisotope émetteur de rayons γ détectable par scintigraphie.

3. Composition selon la revendication 2, caractérisée en ce que le radioisotope est choisi dans le groupe formé par le technétium 99, l'indium 111, l'iode 123 et l'iode 131.

4. Composition selon la revendication 2 ou la revendication 3, caractérisée en ce que le radioisotope est mis en oeuvre à raison de 1 à 5 microcuries par microgramme d'hyaluronectine.

5. Composition selon l'une quelconque des revendications 2 à 4, caractérisée en ce que le radioisotope est l'indium 111 ou le technétium 99, fixé sur l'hyaluronectine par l'intermédiaire d'un agent chélatant, l'acide diéthylènetriamine pentaacétique.

6. Composition selon l'une quelconque des revendications 2 à 4, caractérisée en ce que le radioisotope est l'iode 123 ou l'iode 131, fixé sur l'hyaluronectine au moyen d'iodogène.

7. Composition selon la revendication 1, utilisable pour le traitement, notamment du cancer, caractérisée en ce que l'élément fixé sur l'hyaluronectine est un radioisotope émetteur de rayons β.

8. Composition selon la revendication 7, caractérisée en ce que le radioisotope est l'iode 131.

9. Composition selon la revendication 7 ou la revendication 8, caractérisée en ce que le radioisotope est mis en oeuvre à des doses permettant de délivrer à la tumeur une dose de quelques dizaines de grays.

10. Composition selon la revendication 1, utilisable pour le diagnostic, notamment du cancer, caractérisée en ce que l'élément radioactif est remplacé par un élément paramagnétique détectable par résonance magnétique nucléaire.

## Claims

1. Composition for the diagnostic and/or the treatment, especially of cancer, characterized in that it is consisting of an aqueous injectable solution of a glycoprotein, hyaluronectin, on which a radioactive element is fastened.

2. Composition according to claim 1 for the diagnostic, especially of cancer, characterized in that the radioactive element fastened to the hyaluronectin is a radioisotope emitter of radiation, detectable by scintigraphy.

3. Composition according to claim 2, characterized in that the radioisotope is one of the group formed by technetium 99, indium 111, iodine 123 and iodine 131.

4. Composition according to claim 2 or claim 3, characterized in that the radioisotope is used at the rate of 1 to 5 microcurie per microgram of hyaluronectin.

5. Composition according to one of claims 2 to 4, characterized in that the radioisotope is indium 111 or technetium 99, fastened to hyaluronectin by aid of a chelating agent, the diethylenetriamine pentaacetic acid.

6. Composition according to one of claims 2 to 4, characterized in that the radioisotope is iodine 123 or iodine 131, fastened to hyalyronectin by aid of iodogen.

7. Composition according to claim 1 for the treatment, especially of cancer, characterized in that the element fastened to hyaluronectin is a radioisotope emitter of β-radiation.

8. Composition according to claim 7, characterized in that the radioisotope is iodine 131.

9. Composition according to claim 7 or claim 8, characterized in that the radioisotope is used at rates permitting to deliver to the tumour a dose of some tens grays.

10. Composition according to claim 1 for the diagnostic, especially of cancer, characterized in that the radioactive element is replaced by a paramagnetic element detectable by nuclear magnetic resonance.

## Patentansprüche

1. Zusammensetzung für die Diagnose und/oder die Behandlung, im besonderen von Krebs, dadurch gekennzeichnet, daß sie aus einer injizierbaren wässerigen Lösung eines Glykoproteins, Hyaluronektin, auf welchem ein radioaktives Element befestigt ist, besteht.

2. Zusammensetzung nach Anspruch 1 für die Diagnose, im besonderen von Krebs, dadurch gekennzeichnet, daß das radioaktive, auf dem Hyaluronektin befestigte Element ein γ-Strahlen emittierendes, durch Szintigraphie auffindbares Radio-isotop ist.

3. Zusammensetzung nach Anspruch 2, dadurch gekennzeichnet, daß das Radioisotop Technetium 99, Indium 111, Jod 123 oder Jod 131 ist.

4. Zusammensetzung nach Anspruch 2 oder Anspruch 3, dadurch gekennzeichnet, daß das Radioisotop im Verhältnis von 1 bis 5 Mikrocurie pro Mikrogramm Hyaluronektin benutzt ist.

5. Zusammensetzung nach einem der Ansprüche 2 bis 4, dadurch gekennzeichnet, daß das Radioisotop Indium 111 oder Technetium 99 ist, und daß es auf dem Hyaluronektin über einen chelatbildenden Stoff, die Diethylentriamin pentaessigsäure befestigt ist.

6. Zusammensetzung nach einem der Ansprüche 2 bis 4, dadurch gekennzeichnet, daß das Radioisotop Jod 123 oder Jod 131 ist, und daß es auf dem Hyaluronektin mittels Jodogen befestigt ist.

7. Zusammensetzung nach Anspruch 1 für die Behandlung, im besonderen von Krebs, dadurch gekennzeichnet, daß das auf dem Hyaluronektin befestigte Element ein β-Strahlen emittierendes Radioisotop ist.

8. Zusammensetzung nach Anspruch 7, dadurch gekennzeichnet, daß das Radioisotop Jod 131 ist.

9. Zusammensetzung nach Anspruch 7 oder Anspruch 8, dadurch gekennzeichnet, daß das Radioisotop in Mengen benutzt ist, die es erlauben, dem Tumor eine Dosis von einigen zehn Gray zu liefern.

10. Zusammensetzung nach Anspruch 1 für die Diagnose, im besonderen von Krebs, dadurch gekennzeichnet, daß das radioaktive Element durch ein paramagnetisches, durch magnetische Kernresonanz auffindbares Element ersetzt ist.
